Europäisches Patentamt

European Patent Office

Office européen des brevets

(19)

(11) Publication number: **0 464 208 A1**

## EUROPEAN PATENT APPLICATION
### published in accordance with Art. 158(3) EPC

(12)

(21) Application number: **90915988.1**

(22) Date of filing: **19.01.90**

(86) International application number:
**PCT/SU90/00018**

(87) International publication number:
**WO 91/10469 (25.07.91 91/17)**

(51) Int. Cl.5: **A61M 27/00**

(43) Date of publication of application:
**08.01.92 Bulletin 92/02**

(84) Designated Contracting States:
**BE CH DE FR GB IT LI SE**

(71) Applicant: **POLTAVSKY MEDITSINSKY STOMATOLOGICHESKY INSTITUT**
**ul. Shevchenko, 23,**
**Poltava 314000(SU)**

(72) Inventor: **MAZURIK, Sergei Mikhailovich**

**ul. Lenina, 92-57**
**Poltava, 314022(SU)**
Inventor: **GRITSENKO, Nikolai Ivanovich**
**ul. Khalturina, 21-6**
**Poltava, 314038(SU)**

(74) Representative: **Lerwill, John et al**
**A.A. Thornton & Co. Northumberland House**
**303-306 High Holborn**
**London, WC1V 7LE(GB)**

(54) **T-SHAPED DRAINAGE TUBE.**

(57) The invention relates to medical techniques. A T-shaped drainage tube comprises a longitudinal section (1) and a transverse sectin (2). To reduce traumatization of the common bile duct when removing the tube during the post-operative period, the transverse section (2) consists, according to the invention, of a strip rolled in the form of a three-dimensional cylindrical spiral. The drainage tube is intended for use in hospitals dealing with bile duct surgery.

## Technical Field

The invention relates generally to medical engineering and more specifically to T-drain tubes made use of for drainage of the common bile duct.

## Prior Art

One state-of-the-art T-drain tube is known to be made of an elastic material (latex) and applied for external drainage of extrahepatic bile ducts within the postoperative period (T-Drain, Cattell, code No.2575, product of the USA available from Inmed corporation (2950 Pacific Drive), Norcross, GA, 30071).

The drain tube in question is composed of a longitudinal and a transverse member made as an integral unit of form a T-structure. In the course of surgery the transverse member of said drain tube is situated in the common bile duct, while its longitudinal member is brough outside through a special incision made in the anterior abdominal wall. Within the postoperative period the drain tube under consideration is withdrawn from the common bile duct in such a manner that the double-lumen transverse member folded together is passed through an opening established round a single-lumen member of the T-drain tube, with the result that the walls of the common bile duct is slightly torn or lacerated and a pronounced traumatic injury to the surrounding soft tissue ensues. Moreover, it is through the gaping wound of the common bile duct that a great amount of bile is free to discharge after withdrawn of the drain tube, which might be causative of diverse complications.

In this respect surgeons are to place limitation upon indication for application of drainage of the common bile duct through the use of a known T-drain tube, which is in fact the optimum drainage system applied to the extrahepatic bile ducts.

## Disclosure of the Invention

It is therefore a primary and essential object of the invention to provide a T-drain tube whose construction would enable one to avoid injury to the wall of the common bile duct and to the surrounding tissues when withdrawing the drain tube from the common bile duct within the postoperative period.

The essence of the invention resides in that in a T-drain tube having a transverse portion and a longitudinal portion, both made as an integral unit, according to the invention, the transverse portion is established by a band coiled into a helical coil.

The T-drain tube, according to the present invention, prevents injury to the walls of the common bile duct and to the surrounding soft tissues when being withdrawn from the common bile duct within the postoperative period. Production of the proposed T-drain tubes is less expensive than production of the heretofore-known drain tubes.

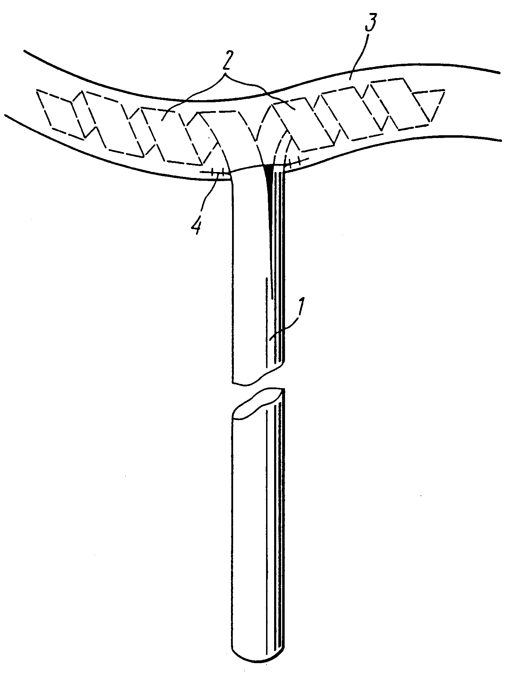

## Summary of the Drawing

In what follows the invention is illustrated by a detailed description of a specific exemplary embodiment thereof with reference to the accompanying drawing, which presents a general perspective view of a T-drain tube, according to the invention, when iserted in the common bile duct.

## Preferred Embodiment of the Invention

The T-drain tube as shown in the Drawing comprises a longitudinal portion 1 and a transverse portion 2, both of them being made as an integral unit. The longitudinal portion 1 is a solid piece shaped as a conventional single-lumen tube, while the transverse portion 2 is established by a band coiled into a cylindrical coil. The bore (lumen) diameters of the longitudinal portion 1 and of the transverse portion 2 are equal to each other, while said portions 1 and 2 make up an angle of 90 degrees with each other.

The T-drain tube of the invention is made of a conventional material made use of for drain tubes, e.g., latex. To this end, a single-lumen drain tube is cut longitudinally into two equal parts for a length of a few centimeters and the resultant strips are wound as a helical coil onto a metal rod of a required diameter, one strip being wound clockwise, while the other, counterclockwise. Then the tube and the rod are immersed in boiling water for a while, whereupon they are let cool down at one and the metal rod is extracted, and the strips acquire a helical shape and establish the transverse tube portion 2, whereas the solid single-lumen part of the tube defines the longitudinal tube portion 1.

The proposed T-drain tube is used as follows.

Once the common bile duct has been examined and it has been found necessary to apply external drainage thereto, the drain tube of the invention is inserted into the choledochotomy opening, while the transverse portion 2 is left in the common bile duct 3, which is stitched up to suit the dimensions of the longitudinal tube portion 1. The drain tube may be fixed with a catgut suture 4 at the place of its entrance into the common bile duct 3. The drain tube is removed within the postoperative period according to positive indications as follows when pulled at the longitudinal portion 1 the tube is made to advance (downwards as shown in the Drawing), while the coil-shaped transverse portion 2 gets unwound to resume the striplike shape, thus avoiding completely the danger of injuring the walls of the common bile duct 3 and the surrounding soft tissues when withdrawing the T-drain tube therefrom. Indications for use of the proposed atraumatic drainage differ in nothing from those for use of conventional Kerr's T-tube drainage.

Industrial Applicability

The invention is applicable in hospitals involved in surgery of bile ducts.

**Claims**

1.  A T-drain tube, comprising a longitudinal portion (1) and a transverse portion (2) made as an integral unit, characterized in that the transverse portion is established by a band coiled into a cylindrical coil.

# INTERNATIONAL SEARCH REPORT

International Application No PCT/SU 90/00018

| I. CLASSIFICATION OF SUBJECT MATTER (If several classification symbols apply, indicate all) [6] |
|---|
| According to International Patent Classification (IPC) or to both National Classification and IPC |
| Int.Cl.[5]    A 61 M 27/00 |

## II. FIELDS SEARCHED

**Minimum Documentation Searched [7]**

| Classification System | Classification Symbols |
|---|---|
| Int.Cl.[5] | A 61 M 27/00 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | US, A, 4072153 (WILLIAM H. SWARTZ), 7 February 1978, (07.02.78), fig.3, column 2, lines 41,42, column 3, lines 1-5 | 1 |
| A | US, A, 4142528 (JOSEPH G. WHELAN et al.), 6 March 1979 (06.03.79), fig. 1, column 4, lines 38-41, column 5, lines 15-40 | 1 |
| A | SU, A1, 1409285 (S.M. Mazurik et al.), 15 July 1988 (15.07.88), fig.1, column 1, lines 7-15 | 1 |

------------

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 16 January 1991 (16.01.91) | 29 January 1991 (29.01.91) |

| International Searching Authority | Signature of Authorized Officer |
|---|---|
| ISA/SU | |

Form PCT/ISA/210 (second sheet) (January 1985)